# EUROPEAN PATENT APPLICATION

(11) **EP 4 154 937 A1**
(43) Date of publication of application: **29.03.2023**
(21) Application number: 22197171.6
(22) Date of filing: 22.09.2022
(51) Int. Cl.: A61M 39/06, A61M 25/00

(54) **FLOATING HEMOSTASIS VALVE**

(30) Priority: 23.09.2021 US 202163247408 P; 16.08.2022 US 202217888734
(71) Applicant: Biosense Webster (Israel) Ltd, 2066717 Yokneam (IL)
(72) Inventor: TANG, Raymond Yue-Sing, Irvine, 92618 (US)
(74) Representative: Carpmaels & Ransford LLP

(57) **Abstract**

Disclosed herein is a system for treatment including a sheath introducer and a catheter or other device. The system can have a floating hemostasis valve comprising a seal through which the shaft of the catheter can extend. The floating hemostasis valve can have elastic bellows which can allow the seal to move orthogonal to the longitudinal axis of the sheath introducer in response to an orthogonal movement of the shaft of the catheter. The elastic bellows can be configured to deform to allow the orthogonal movement of the floating hemostasis valve.

## Description

### CROSS-REFERENCE TO RELATED APPLICATION

The present application claims priority to prior filed U.S. Provisional Application No. 63/247,408 filed on September 23, 2021, which is hereby incorporated by reference as set forth in full herein.

### FIELD

The present invention relates to valves for catheter delivery, and in particular hemostasis valves for a sheath introducer.

### BACKGROUND

A "sheath" or "sheath introducer" includes a tube placed in an artery or vein of a patient during a medical procedure that, when positioned for treatment, has a distal end within the artery or vein and a proximal end outside of the patient. A hemostasis valve at the proximal end of the sheath inhibits blood from exiting the sheath and allows longer sheaths and/or catheters to be inserted through the sheath into the artery or vein.

A problem with some current sheath introducers for catheter delivery is that, when retracting devices from the sheath, an off-axis force can disturb the seal of the sheath and potentially cause a valve leak to occur.

### SUMMARY

The present invention relates to valves for catheter delivery, and in particular hemostasis valves for a sheath introducer.

An example system is disclosed herein for intravascular treatment including a sheath introducer and a catheter or other device. The sheath can have a floating hemostasis valve comprising a seal through which the shaft of the catheter can extend. The floating hemostasis valve can have elastic bellows which can allow the seal to move orthogonal to the longitudinal axis of the sheath introducer in response to an orthogonal movement of the shaft of the catheter. The elastic bellows can be configured to deform to allow the orthogonal movement of the floating hemostasis valve.

An example handle is disclosed herein configured for a sheath introducer. The handle i can include an elongated handle housing; an elongated shaft disposed within the elongated handle housing and comprising a lumen concentric to a longitudinal axis of the handle; and a seal comprising an opening centered with the longitudinal axis, the seal being constrained to move orthogonally in relation to the longitudinal axis in response to a force along a longitudinal axis applied to the seal.

In any of the examples disclosed herein, the handle can further have a deformable annulus comprising an inner perimeter fixed in relation to the seal and an outer perimeter fixed in relation to the elongated handle housing, the deformable annulus being configured to deform in response the force applied to the seal.

In any of the examples disclosed herein, the deformable annulus can comprise bellows.

In any of the examples disclosed herein, the bellows can include a circular ridge concentric to the longitudinal axis and constrained to move orthogonally in relation to the longitudinal axis in response the force applied to the seal.

In any of the examples disclosed herein, the handle can further have a rigid annulus affixed to the seal and affixed to the inner perimeter of the deformable annulus, the rigid annulus being constrained to move orthogonally in relation to the longitudinal axis in response the force applied to the seal.

In any of the examples disclosed herein, the rigid annulus can include a proximal portion and a distal portion such that an outer perimeter of the seal is positioned between the proximal portion and the distal portion.

In any of the examples disclosed herein, the deformable annulus can include an inner perimeter positioned between the proximal portion of the rigid annulus and the distal portion of the rigid annulus and an outer perimeter fixed in relation to the elongated handle housing.

In any of the examples disclosed herein, the handle can further have a proximal housing portion including an annular proximal surface defining a proximal end of the elongated handle housing, the proximal housing portion comprising a circular opening concentric with the longitudinal axis.

In any of the examples disclosed herein, the elongated handle housing can include a proximal portion configured to inhibit proximal movement of the seal and a distal portion configured to inhibit distal movement of the seal. Each of the proximal and distal portions respectively can have a circular opening and a tubular extension extending distally from the respective circular opening. Each of the circular openings and the tubular extensions can be concentric to the longitudinal axis.

In any of the examples disclosed herein, the handle can further have a rigid annulus affixed to the seal. The rigid annulus can be constrained to move orthogonally in relation to the longitudinal axis in response the force applied to the seal. The rigid annulus can include a proximal surface configured to slide orthogonally against the proximal portion of the elongated handle housing in response the force applied to the seal. The rigid annulus can include a distal surface configured to slide orthogonally against the distal portion of the elongated handle housing in response the force applied to the seal.

An example sheath is disclosed herein that can be configured for insertion into a blood vessel and/or artery. The sheath can include an elongated shaft having a lumen concentric to a longitudinal axis of the sheath. The sheath can include a seal approximate a proximal end of the elongated shaft including an opening concentric to the longitudinal axis. The seal can be constrained to move orthogonally in relation to the longitudinal axis in response to a force along a longitudinal axis applied to the seal. The sheath can include a fluid impermeable assembly circumscribing an outer perimeter of the seal and circumferentially sealing to the lumen of the elongated shaft.

In any of the examples disclosed herein, the fluid impermeable assembly can have a deformable annulus comprising an inner perimeter fixed in relation to the seal and an outer perimeter fixed in relation to the longitudinal axis. The deformable annulus cab be configured to deform in response the force applied to the seal.

In any of the examples disclosed herein, the deformable annulus can include bellows.

In any of the examples disclosed herein, the bellows can include a circular ridge concentric to the longitudinal axis and constrained to move orthogonally in relation to the longitudinal axis in response the force applied to the seal.

In any of the examples disclosed herein, the sheath can further have a rigid annulus affixed to the seal and affixed to the inner perimeter of the deformable annulus. The rigid annulus can be constrained to move orthogonally in relation to the longitudinal axis in response the force applied to the seal.

In any of the examples disclosed herein, the rigid annulus can have a proximal portion and a distal portion such that an outer perimeter of the seal is positioned between the proximal portion and the distal portion.

In any of the examples disclosed herein, the deformable annulus can include an inner perimeter and an outer perimeter. The inner perimeter can be positioned between the proximal portion and the distal portion of the rigid annulus. The inner perimeter can be fixed in relation to the seal. The outer perimeter of the deformable annulus can be fixed in relation to the longitudinal axis.

In any of the examples disclosed herein, the sheath can further have an annular proximal surface defining a proximal end of the sheath. The annular proximal surface can include a circular opening concentric with the longitudinal axis.

In any of the examples disclosed herein, the sheath can further include a proximal sheath portion configured to inhibit proximal movement of the seal and fixed in relation to the longitudinal axis and a distal sheath portion configured to inhibit distal movement of the seal and fixed in relation to the longitudinal axis. Each of the proximal and distal sheath portions can respectively include a circular opening and a tubular extension extending distally from the respective circular opening. Each of the circular openings and the tubular extensions can be concentric to the longitudinal axis.

In any of the examples disclosed herein, the sheath can further include a rigid annulus affixed to the seal. The rigid annulus can be constrained to move orthogonally in relation to the longitudinal axis in response the force applied to the seal. The rigid annulus can include a proximal surface configured to slide orthogonally against the proximal sheath portion in response the force applied to the seal. The rigid annulus can have a distal surface configured to slide orthogonally against the distal sheath portion in response the force applied to the seal.

An example method of treatment is disclosed herein which includes the steps of inserting a tubular device through a seal of a hemostasis valve of a sheath introducer; moving the tubular device to an angle of about 5 degrees from the longitudinal axis as measured on a proximal side of the seal; and maintaining hemostasis between the seal and the tubular device while the tubular device is at the angle of about 5 degrees.

In any of the examples disclosed herein, the method can further include moving the tubular device to apply a force along a longitudinal axis to the seal thereby translating the seal in an orthogonal direction, the orthogonal force and the orthogonal direction each being orthogonal to a longitudinal axis of the sheath introducer.

In any of the examples disclosed herein, the method can further include moving the tubular device to apply the orthogonal force, thereby deforming a deformable annulus comprising an inner perimeter fixed in relation to the seal an outer perimeter fixed in relation to the longitudinal axis.

In any of the examples disclosed herein, deforming the deformable annulus can include compressing bellows of the deformable annulus.

In any of the examples disclosed herein, deforming the deformable annulus can comprise moving a circular ridge of the bellows in the orthogonal direction.

In any of the examples disclosed herein, the method can further include translating, in the orthogonal direction, a rigid annulus affixed to the seal and affixed to the inner perimeter of the deformable annulus.

In any of the examples disclosed herein, the method can further include inserting the tubular device through a circular opening at a proximal end of the sheath introducer, the circular opening being concentric with the longitudinal axis.

These and other aspects of the disclosed technology are described herein along with the accompanying figures. Other aspects, features, and elements of the disclosed technology will become apparent to those skilled in the pertinent art upon reviewing the following description of specific examples of the disclosed technology. While features of the disclosed technology may be discussed relative to certain examples and figures, the disclosed technology can include one or more of the features or elements discussed herein. Further, while one or more examples may be discussed as having certain advantageous features, one or more of such features may also be used with the various other examples of the disclosure discussed herein. In similar fashion, while certain examples, implementations, and embodiments may be discussed below with respect to a given device, system, or method, it is to be understood that such examples can be implemented in various other devices, systems, and methods of the present disclosure.

### BRIEF DESCRIPTION OF THE DRAWINGS

Figure 1 illustrates a system for intralumenal treatment including a sheath having a floating hemostasis valve according to aspects of the present invention.
Figure 2 illustrates a treatment step utilizing the sheath having the floating hemostasis valve according to aspects of the present invention.
Figure 3A illustrates a cross-section of the hemostasis valve centered on a longitudinal axis of the sheath according to aspects of the present invention.
Figure 3B illustrates a cross-section of the hemostasis valve shifted orthogonal to the longitudinal axis of the sheath according to aspects of the present invention.
Figures 4A through 4D illustrate close-up of the cross sections of the hemostasis valve as indicated in Figures 3A and 3B. Subsection A-A is illustrated in FIG. 4A, subsection B-B is illustrated in FIG. 4B, subsection C-C is illustrated in FIG. 4C, and subsection D-D is illustrated in FIG. 4D.
Figure 5 is an exploded view of the floating hemostasis valve according to aspects of the present invention.
Figure 6 illustrates a system for intralumenal treatment including a sheath having a floating hemostasis valve according to aspects of the present invention.
Figure 7 is a flow diagram illustrating a method for using a floating hemostasis valve according to aspects of the present invention.

### DETAILED DESCRIPTION

The present disclosure can provide a floating valve for use in sheath introducers. The valve can be centered on the longitudinal axis of the sheath introducer thus allowing for devices to be inserted and/or retracted at an angle without causing a partial opening of the valve. A problem with some current sheath introducers for catheter delivery is that, when retracting devices from the sheath, an off-axis force can disturb the seal of the sheath and potentially cause a valve leak to occur. This is, at least partially, due to the fact that current sheath introducers have a fixed valve centered on the longitudinal axis of the sheath. Examples presented herein may mitigate valve leakage by including the floating valve.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 99%.

As used herein, the terms "comprising" or "containing" or "including" are interpreted to mean that at least the named compound, element, particle, or method step is present in the composition or article or method, but does not exclude the presence of other compounds, materials, particles, method steps, even if the other such compounds, material, particles, method steps have the same function as what is named.

As used herein, the use of terms such as "having," "has," "including," or "includes" are open-ended and are intended to have the same meaning as terms such as "comprising" or "comprises" and not preclude the presence of other structure, material, or acts. Similarly, though the use of terms such as "can" or "may" are intended to be open-ended and to reflect that structure, material, or acts are not necessary, the failure to use such terms is not intended to reflect that structure, material, or acts are essential. To the extent that structure, material, or acts are presently considered to be essential, they are identified as such.

As used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

Ranges described as being between a first value and a second value are inclusive of the first and second values, as well as all values therebetween. Likewise, ranges described as being from a first value and to a second value are inclusive of the first and second values, as well as all values therebetween.

It is also to be understood that the mention of one or more method steps does not preclude the presence of additional method steps or intervening method steps between those steps expressly identified.

The components described hereinafter as making up various elements of the disclosure are intended to be illustrative and not restrictive. Many suitable components that would perform the same or similar functions as the components described herein are intended to be embraced within the scope of the disclosure. Such other components not described herein can include, but are not limited to, for example, similar components that are developed after development of the presently disclosed subject matter.

Reference will now be made in detail to examples of the disclosed technology, such as those illustrated in the accompanying drawings. Wherever convenient, the same references numbers will be used throughout the drawings to refer to the same or like parts.

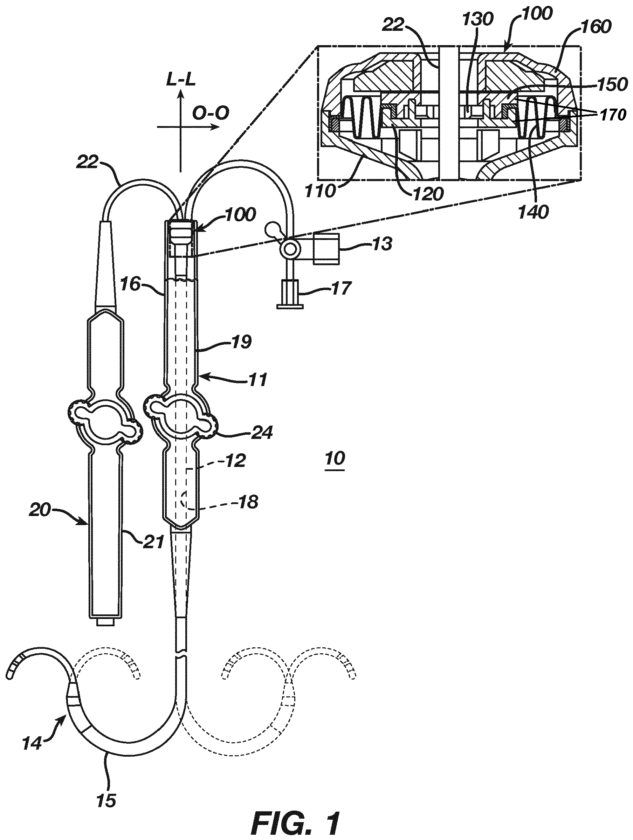

Figure 1 illustrates a system 10 for intralumenal treatment including a sheath introducer 11 and a catheter or other percutaneous device 20. The sheath introducer includes an example floating hemostasis valve assembly 100. The sheath introducer 11 can include an elongated shaft 12 and a control handle 16. The control handle 16 can include a generally elongated handle housing 19 and a deflection knob 24. The elongated shaft 12 and handle housing 19 can be aligned along a longitudinal axis L-L. The shaft 12 enters the control handle 16 at its distal end, extends along the longitudinal axis L-L of the control handle 16 and terminates near a proximal end of the control handle at the floating hemostasis valve 100. The floating hemostasis valve 100 forms a fluid tight seal with a shaft 22 of the device 20 for various purposes, including keeping a lumen 18 of the shaft 12 at a positive pressure to prevent patient's loss of blood through the sheath introducer 11 and minimizing the introduction of air in the patient's body. Moreover, the floating hemostasis valve 100 can connect to a side port 13 having a luer hub 17 through which a vacuum can be created to remove air from the inner lumen 18 or through which fluids can be flushed into the lumen 18 to prevent blood from clotting.

The floating hemostasis valve 100 can include a seal 130 through which the shaft 22 of the other device 20 extends. An outer perimeter of the seal 130 can be secured by a rigid annulus having a proximal portion 150 and a distal portion 120. An inner perimeter of a deformable annulus 140 can be affixed to the rigid annulus (e.g., secured between the proximal portion 150 and distal portion 120 as illustrated). An outer perimeter of the elastic deformable annulus 140 can be affixed to the housing 19 of the handle 16. As illustrated in the enlarged view of the valve 100, the housing 19 can include a proximal portion 160 and a distal hub portion 110 around the seal 130, rigid annulus 150, 120, and deformable annulus 140. The seal 130 and the proximal portion 150 and distal portion 120 of the rigid annulus can move orthogonal to the longitudinal axis L-L of the control handle 16 in response to orthogonal movement of the shaft 22 of the other device 20. The elastic deformable annulus 140 can be configured to deform to allow the orthogonal movement of the valve 130 and rigid annulus of portions 120, 150.

In use, the shaft 12 of the sheath introducer 11 is introduced into a patient's body through an opening in a vein. A guidewire (not illustrated) can be fed through the lumen 18 of the sheath introducer 11, as understood by a person skilled in the pertinent art. In some treatments the guidewire can be followed by a dilator. The dilator can be removed. The device 20 can be introduced through the floating hemostasis valve 100 at the proximal end of the control handle 16 to enter the central lumen 18 of the introducer 11 whereby the guidewire is passed through a guidewire lumen in the device 20. For devices not containing a guidewire lumen, the guidewire can be removed from the sheath prior to insertion of the device. The luer hub 17 on the side port 13 can be used to draw or inject fluid into the central lumen 18 of the sheath introducer 11 as needed.

The sheath introducer 11 can include a deflectable distal section 14 as illustrated in Figure 1 or otherwise configured as understood by a person skilled in the pertinent art. When the sheath introducer 11 is deflectable, an electrophysiologist can use one hand to manipulate the control handle 16 of the sheath introducer 11 and his other hand to manipulate the control handle 21 of the device 20. The electrophysiologist can deflect the deflectable region 15 of the shaft 12 with one hand while deflecting an exposed deflectable region of the device 20 with the other hand. The sheath introducer 11 can otherwise be configured similarly to as disclosed in U.S. Patent No. 10,046,141, incorporated herein by reference, and attached in the Appendix to priority application U.S. 63/247,408.

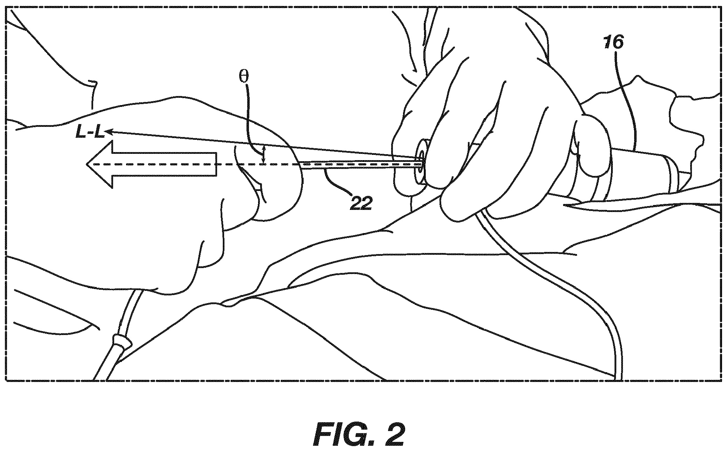

Figure 2 illustrates a treatment step utilizing the sheath introducer 11 having the floating hemostasis valve 100. As shown, the control handle 16 can include a generally elongated handle housing aligned along a longitudinal axis L-L. The shaft 12 enters the control handle 16 at its distal end, extends along the longitudinal axis L-L of the control handle 16 and terminates near a proximal end of the control handle at the floating hemostasis valve 100. The shaft 22 of other device 20 can also enter and exit the control handle 16 at the proximal end. During manipulation of the other device 20, the shaft 22 of the other device may be moved by the physician to an angle θ in relation to the longitudinal axis L-L. In some examples presented herein, the shaft 22 of the other device 20 can be positioned in the control handle 16 an angle θ of about 5 degrees from the longitudinal axis L-L while maintaining a hemostatic seal between the hemostatic valve 100 and the shaft 22 of the other device 20. The seal 130 and rigid annulus 170 can move orthogonal to the longitudinal axis L-L of the control handle 16 in response to orthogonal movement of the shaft 22 of the other device 20. In such a manner, the control handle 16 can maintain hemostasis between the seal 130 and the shaft 22 of other device 20 while the shaft 22 of other device 20 is at the angle of about 5 degrees.

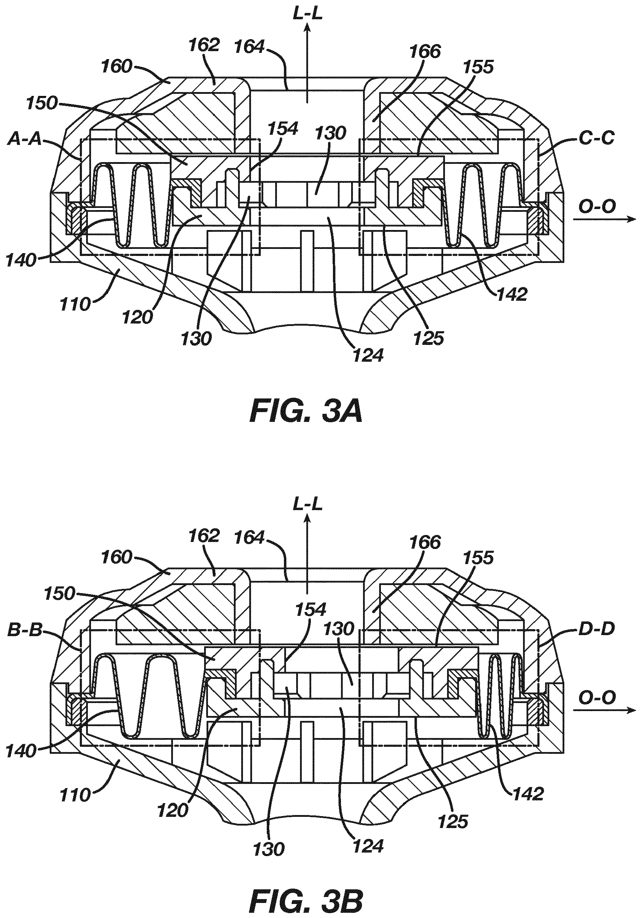

Figure 3A illustrates a cross-section of the hemostasis valve 100 centered on the longitudinal axis L-L, positioned similarly to as shown in FIG. 1.

Figure 3B illustrates a cross-section of the hemostasis valve 100 in which the seal 130 is moved in an orthogonal direction O-O to the longitudinal axis L-L.

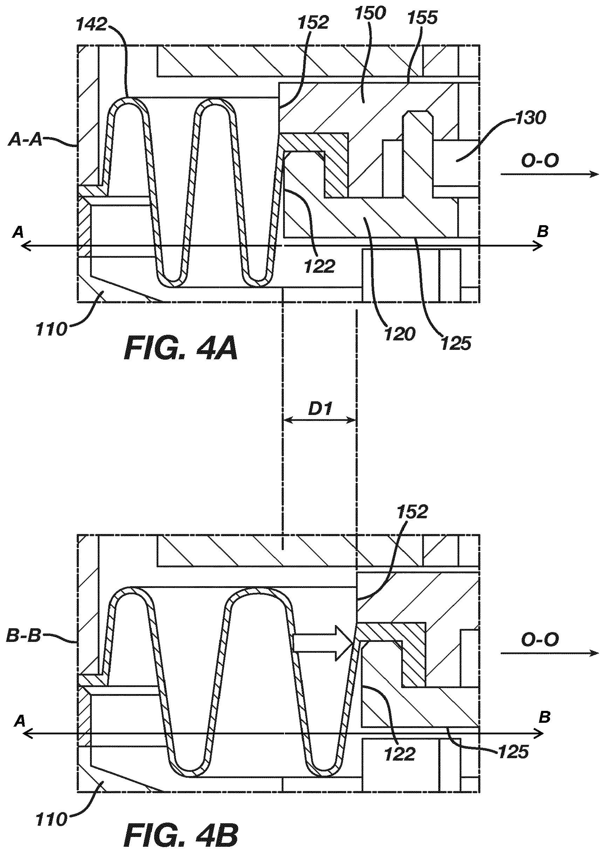

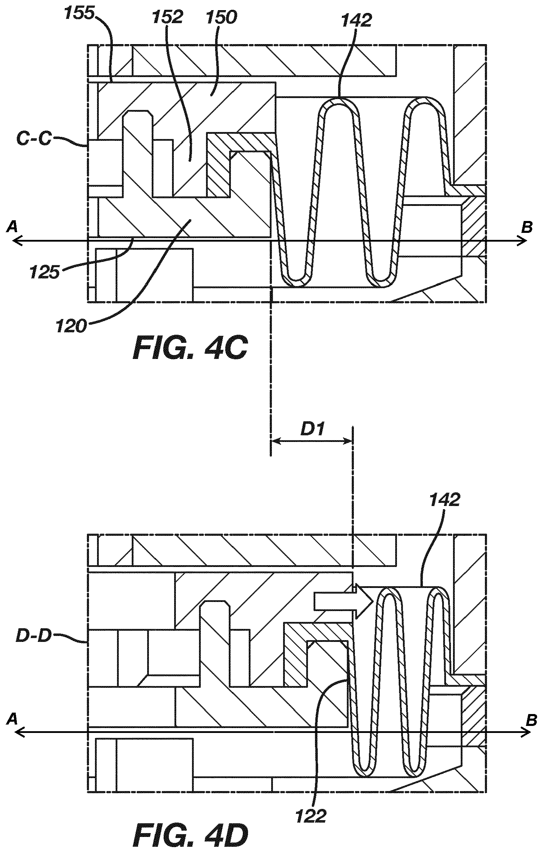

Figures 4A through 4D illustrate close-up of the cross sections of the hemostasis valve assembly 100 as indicated in Figures 3A and 3B. Subsection A-A is illustrated in FIG. 4A, subsection B-B is illustrated in FIG. 4B, subsection C-C is illustrated in FIG. 4C, and subsection D-D is illustrated in FIG. 4D.

Referring collectively to Figures 3A, 3B, and 4A through 4D, the distal portion 120 of the rigid annulus can have an inner portion 124 affixed to the seal 130, an outer portion 122 affixed to the deformable annulus 140, and a distal surface 125 configured to slide against the hub 110 of the handle housing 19. Similarly, the proximal portion 150 of the rigid annulus can have an inner portion 154 affixed to the seal 130, an outer portion 152 affixed the deformable annulus 140, and a proximal surface 155 configured to slide against the proximal portion 160 of the handle housing 19. The rigid annulus can be constrained to move orthogonally in relation to the longitudinal axis L-L in response to a force along a longitudinal axis applied to the seal 130.

The proximal portion 160 of the handle housing 19 can have a proximal surface 162, an opening 164, and a tubular extension 166. The proximal surface 162 can define a proximal end of the elongated handle housing 19, and the opening 164 in the proximal portion 160 can be a circular opening concentric with the longitudinal axis L-L. The proximal portion 160 can inhibit proximal movement of the seal 130.

The deformable annulus 140 can include baffles or bellows 142. The deformable annulus can include an inner perimeter 144 fixed in relation to the seal 130 and an outer perimeter 148 fixed in relation to the elongated handle housing 19. The deformable annulus 140 can be configured to deform in response to a force along a longitudinal axis applied to the seal 130. The bellows can comprise a circular ridge concentric to the longitudinal axis L-L, and the ridges can be constrained to move orthogonally in relation to the longitudinal axis L-L in response to a force along a longitudinal axis applied to the seal 130.

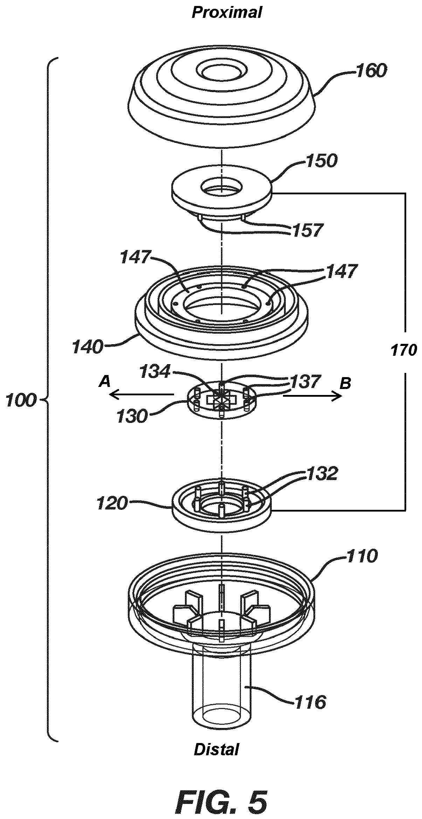

As shown in Figure 3B, the rigid annulus 170 (Fig. 5) represented by 120 and 150 can be affixed to the seal 130 and constrained to move orthogonally in relation to the longitudinal axis L-L in response the force applied to the seal 130. The proximal surface 155 is constrained to slide orthogonally against the proximal portion 160 of the elongated handle housing 19 in response the force applied to the seal 130. The distal surface 125 is constrained to slide orthogonally against the distal portion of the elongated handle housing 19 in response the force applied to the seal 130.

As shown in Figures 4A through 4D, the baffles 142 of the deformable annulus 140 can be configured to deform to allow the orthogonal movement of the seal 130 along the orthogonal axis O-O. During such an orthogonal movement on a plane defined by arrows A and B, the seal 130 can pull the distal portion 120 and the proximal portion 150 of the rigid annulus, which is attached thereto. The outer perimeter 122 of the distal portion 120 and the outer perimeter 152 of the proximal portion 150 can be affixed to the deformable annulus 140 and translate the movement along the orthogonal axis O-O from the seal 130 to the deformable annulus 140. Such movement is indicated by the first distance D1. As shown, when the deformable annulus 140 expands the first distance D1, the baffles 142 can also expand to maintain contact with the rigid annulus 170 (shown as portions 120, 150) and portions 160, 110 of the handle housing 19.

Consequently, when a force is applied to seal 130 at an angle to longitudinal axis L-L (or parallel but off-centered to L-L), seal 130 is constrained from tilting with respect to the longitudinal axis L-L. Seal 130 therefore can only translate on the plane generally defined by arrows A and B. Seal 130 pulling on the deformable annulus 140 on a first side of the seal 130 (during translation on plane A-B) can have an equal and opposite effect on the deformable annulus on a second side of the seal 130 opposite the first side, as shown in FIGs. 4C and 4D. The seal 130 can push on the proximal portion 150 and the distal portion 120 of the rigid annulus, which in turn can compress the deformable annulus 140. In response, the baffles 142 can collapse to accommodate such a movement. The compression of the deformable annulus 140 can be equal to the first distance D1 at which the deformable annulus 140 compresses in FIGs. 4A and 4B.

Figure 5 is an exploded view of the floating hemostasis valve assembly 100 showing the proximal portion 160 of the handle housing 19, the proximal portion 150 of the rigid annulus 170, the deformable annulus 140, the seal 130, the distal portion 120 of the rigid annulus 170, and the hub 110 on the distal portion of the handle housing 19. Some or all components illustrated in Figure 5 can share a similar longitudinal axis L-L.

The outer perimeter of the seal 130 and the inner perimeter of the deformable annulus 140 can be secured between the proximal portion 150 and the distal portion 120 of the rigid annulus 170. The seal 130 can include a central opening 134 aligned with the longitudinal axis L-L. The seal 130 can also include and holes 137 near the outer perimeter 132 of the seal 130. The distal portion 120 of the rigid annulus can include protrusions 127 or posts that are sized, positioned, and otherwise configured to extend through the holes 137 near the outer perimeter 132 of the seal 130. The deformable annulus 140 can include holes 147 near the inner perimeter 144 of the deformable annulus 140. The proximal portion 150 of the rigid annulus can include protrusions 157 or posts that are sized, positioned, and otherwise configured to extend through the holes 147 near the inner perimeter 144 of the deformable annulus 140. The protrusions 127, 157 on the distal portion 120 and proximal portion 150 of the rigid annulus 170 can also extend to provide a friction fit between the distal portion 120 and the proximal portion 150 when the hemostasis valve assembly 100 is assembled.

From Fig. 5, it is noted that seal 130 is captive between proximal rigid annulus portion 150 and distal rigid annulus portion 120 such that seal 130 is constrained from tilting with respect to the longitudinal axis. Because seal 130 is prevented from tilting (relative to axis L-L) by portions 120 and 150, the only direction that seal 130 can move is any direction at right angle (e.g., arrow A or B) to the longitudinal axis L-L. The bellows 140 and annulus 170 allow the seal 130 to move orthogonally (e.g., on a common plane defined by lines A and B) in the same plane defined by arrows A and B. Consequently, when sheath 22 is retracted out of seal opening 134, the sheath 22 may be off-axis with respect to longitudinal axis L-L, and the entire seal 130 is configured to move orthogonally with respect to axis L-L to accommodate the off-axis sheath 22 without leaking back through the seal opening 134. Similarly, when sheath 22 is inserted off-axis into seal opening 134, seal 130 will accommodate the off-axis insertion of sheath 22 without leakage back through the seal opening 134 because the seal 130 is not tilted out of plane and remains in the common plane of lines A and B (while being translated orthogonally).

The outer perimeter 148 of the deformable annulus 140 can be secured to the handle housing 19, between the proximal portion 160 of the handle housing 19 and the distal portion, hub 110 of the handle housing 19. The hub 110 can include a tubular extension 116 to aid in receiving and/or guiding tubular components, such as the sheath 22 of the other device 20.

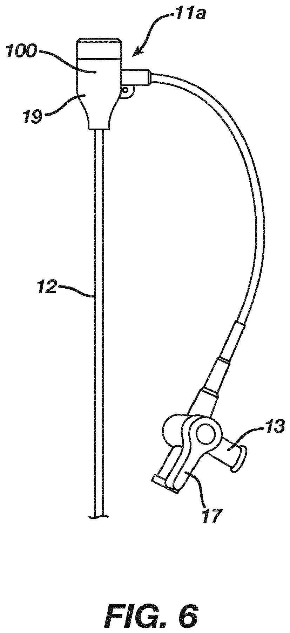

Figure 6 illustrates a system for intralumenal treatment including a sheath introducer 11a. The sheath introducer 11a includes an example floating hemostasis valve 100. The sheath introducer 11a can include an elongated shaft 12 and a control handle housing 19. The control handle housing 19 can include a generally elongated handle housing aligned along a longitudinal axis L-L. The shaft 12 enters the control handle housing 19 at its distal end, extends along the longitudinal axis L-L of the control handle housing 19 and terminates near a proximal end of the control handle at the floating hemostasis valve 100.

In use, the shaft 12 of the sheath introducer 11a is introduced into a patient's body through an opening in a vein. A guidewire can be fed through the lumen of the shaft 12 of the sheath introducer 11a. In some treatments the guidewire can be followed by a dilator, as is generally known in the art. The dilator can be removed. A device, such as the other device 20, can be introduced through the floating hemostasis valve 100 at the proximal end of the control handle housing 19 to enter the central lumen of the sheath introducer 11a whereby the guidewire is passed through a guidewire lumen. For devices not containing a guidewire lumen, the guidewire can be removed from the sheath prior to insertion of the device. The luer hub 17 on the side port 13 can be used to draw or inject fluid into the central lumen of the sheath introducer 11a as needed.

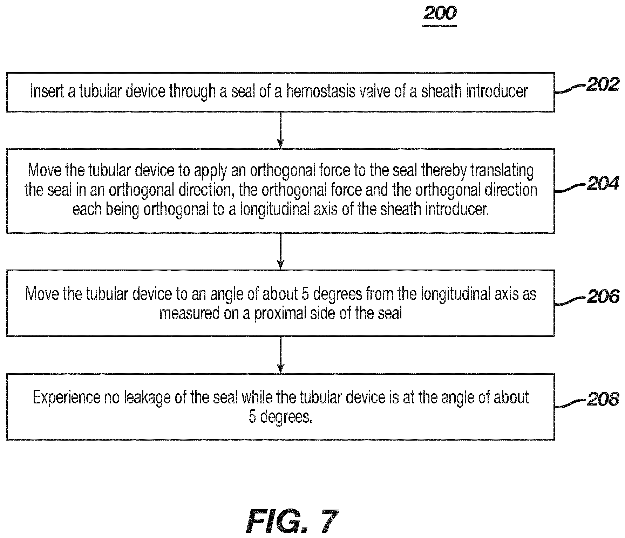

Figure 7 is a flow diagram illustrating a method 200 for using a floating hemostasis valve assembly 100. As shown in block 202, the method 200 can include inserting a tubular device 20 through a seal 130 of a floating hemostasis valve assembly 100 of a sheath introducer 11. The device 20 can be inserted along a longitudinal axis L-L which can be shared with the sheath introducer 11 and the seal 130.

In block 204, the method 200 can include moving the tubular device 20 to apply a force along a longitudinal axis to the seal 130. The orthogonal force can be applied along an orthogonal axis O-O which is orthogonal to the longitudinal axis L-L. The movement can translate the seal 130 in an orthogonal direction along the orthogonal axis O-O which can also be orthogonal to the longitudinal axis L-L.

As shown in block 206, the method 200 can include moving the tubular device 20 to an angle of about 5 degrees from the longitudinal axis L-L. The angle can be measured on a proximal side of the seal 130. Furthermore, as shown in block 208, the method 200 can comprise experiencing no leakage of the seal 130 while the tubular device 22 is at the angle of about 5 degrees.

The descriptions contained herein are examples of embodiments of the invention and are not intended in any way to limit the scope of the invention. As described herein, the invention contemplates many variations and modifications of system components, including alternative combinations of components illustrated in separate figures, alternative materials, alternative component geometries, and alternative component placement. Modifications and variations apparent to those having skilled in the pertinent art according to the teachings of this disclosure are intended to be within the scope of the claims which follow.

## Claims

1. A handle configured for a sheath introducer, the handle comprising:
an elongated handle housing;
an elongated shaft disposed within the elongated handle housing and comprising a lumen concentric to a longitudinal axis of the handle; and
a seal comprising an opening substantially centered with the longitudinal axis, the seal being constrained to translate orthogonally in relation to the longitudinal axis in response to a force applied to the seal along or at an angle to the longitudinal axis.

2. The handle of claim 1, further comprising:
a deformable annulus comprising an inner perimeter fixed in relation to the seal and an outer perimeter fixed in relation to the elongated handle housing, the deformable annulus being configured to deform in response the force applied to the seal.

3. The handle of claim 1, further comprising:
a proximal housing portion comprising an annular proximal surface defining a proximal end of the elongated handle housing, the proximal housing portion comprising a circular opening concentric with the longitudinal axis.

4. The handle of claim 1,
the elongated handle housing comprising a proximal portion configured to inhibit proximal movement of the seal and a distal portion configured to inhibit distal movement of the seal,
each of the proximal and distal portions respectively comprising a circular opening and a tubular extension extending distally from the respective circular opening, and
each of the circular openings and the tubular extensions being concentric to the longitudinal axis.

5. The handle of claim 4, further comprising:
a rigid annulus affixed to the seal, constrained to move orthogonally in relation to the longitudinal axis in response the force applied to the seal, comprising a proximal surface configured to slide orthogonally against the proximal portion of the elongated handle housing in response the force applied to the seal, and comprising a distal surface configured to slide orthogonally against the distal portion of the elongated handle housing in response the force applied to the seal.

6. A sheath configured for insertion into a blood vessel and/or artery, the sheath comprising:
an elongated shaft comprising a lumen concentric to a longitudinal axis of the sheath;
a seal approximate a proximal end of the elongated shaft comprising an opening concentric to the longitudinal axis, the seal being constrained to move orthogonally in relation to the longitudinal axis in response to a force applied to the seal along or at an angle to the longitudinal axis; and
a fluid impermeable assembly circumscribing an outer perimeter of the seal and circumferentially sealing to the lumen of the elongated shaft.

7. The sheath of claim 6, the fluid impermeable assembly comprising:
a deformable annulus comprising an inner perimeter fixed in relation to the seal and an outer perimeter fixed in relation to the longitudinal axis, the deformable annulus being configured to deform in response the force applied to the seal.

8. The handle of claim 2 or the sheath of claim 7, the deformable annulus comprising bellows.

9. The handle or sheath of claim 8, the bellows comprising a circular ridge concentric to the longitudinal axis and constrained to move orthogonally in relation to the longitudinal axis in response the force applied to the seal.

10. The handle of claim 2 or the sheath of claim 7, further comprising:
a rigid annulus affixed to the seal and affixed to the inner perimeter of the deformable annulus, the rigid annulus being constrained to move orthogonally in relation to the longitudinal axis in response the force applied to the seal.

11. The handle or sheath of claim 10, the rigid annulus comprising a proximal portion and a distal portion such that an outer perimeter of the seal is positioned between the proximal portion and the distal portion.

12. The handle of claim 11, the deformable annulus comprising an inner perimeter positioned between the proximal portion of the rigid annulus and the distal portion of the rigid annulus and an outer perimeter fixed in relation to the elongated handle housing.

13. The sheath of claim 11,
the deformable annulus comprising an inner perimeter and an outer perimeter,
the inner perimeter being positioned between the proximal portion and the distal portion of the rigid annulus and being fixed in relation to the seal, and
the outer perimeter of the deformable annulus being fixed in relation to the longitudinal axis.

14. The sheath of claim 6, further comprising:
an annular proximal surface defining a proximal end of the sheath, the annular proximal surface comprising a circular opening concentric with the longitudinal axis.

15. The sheath of claim 6, further comprising:
a proximal sheath portion configured to inhibit proximal movement of the seal and fixed in relation to the longitudinal axis; and
a distal sheath portion configured to inhibit distal movement of the seal and fixed in relation to the longitudinal axis,
each of the proximal and distal sheath portions respectively comprising a circular opening and a tubular extension extending distally from the respective circular opening, and
each of the circular openings and the tubular extensions being concentric to the longitudinal axis.

16. The sheath of claim 15, further comprising:
a rigid annulus affixed to the seal and constrained to move orthogonally in relation to the longitudinal axis in response the force applied to the seal, the rigid annulus comprising a proximal surface configured to slide orthogonally against the proximal sheath portion in response the force applied to the seal and a distal surface configured to slide orthogonally against the distal sheath portion in response the force applied to the seal.
